(19) Europäisches Patentamt
European Patent Office
Office européen des brevets

(11) EP 1 452 533 A1

(12) EUROPEAN PATENT APPLICATION

(43) Date of publication:
01.09.2004 Bulletin 2004/36

(51) Int Cl.7: C07D 401/12, A61K 31/4439, A61P 1/04

(21) Application number: 04004420.8

(22) Date of filing: 26.02.2004

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 28.02.2003 IN MA01562003

(71) Applicant: Dr. Reddy's Laboratories Ltd.
Hyderabad 500 016 (IN)

(72) Inventors:
- Venkatraman, Sundaram
  Hyderabad 500 018 Andhra Pradesh (IN)
- Reddy, Manne Satyanarayana
  ,H.No.8-3-167/D/16
  Hyderabad 500 038 (IN)
- Eswaraiah, Sajja, Lig 110 Dharma Reddy Colony
  Hyderabad 500 072 (IN)
- Bhaskar, Bolugoddu Vijaya
  Hyderabad (IN)
- Reddy, Pingili Ramchandra
  Hyderabad 500 018 Andra Pradesh (IN)
- Rajiv, Ireddy c/o Ramchander, H.No. 5-5-7/27
  Hyderabad 500 072 Andra Pradesh (IN)
- Babu, Thirunava Karasu Ananda
  Thirunagar, Madurai Tamilnadu (IN)

(74) Representative:
Ebner von Eschenbach, Jennifer et al
Ladas & Parry,
Dachauerstrasse 37
80335 München (DE)

# (54) Crystalline form Z of rabeprazole sodium and process for preparation thereof

(57) The present invention relates to novel crystalline Form Z of rabeprazole sodium and a process for the preparation of the crystalline Form Z as well as composition, pharmaceutical composition and method utilizing the crystalline Form Z.

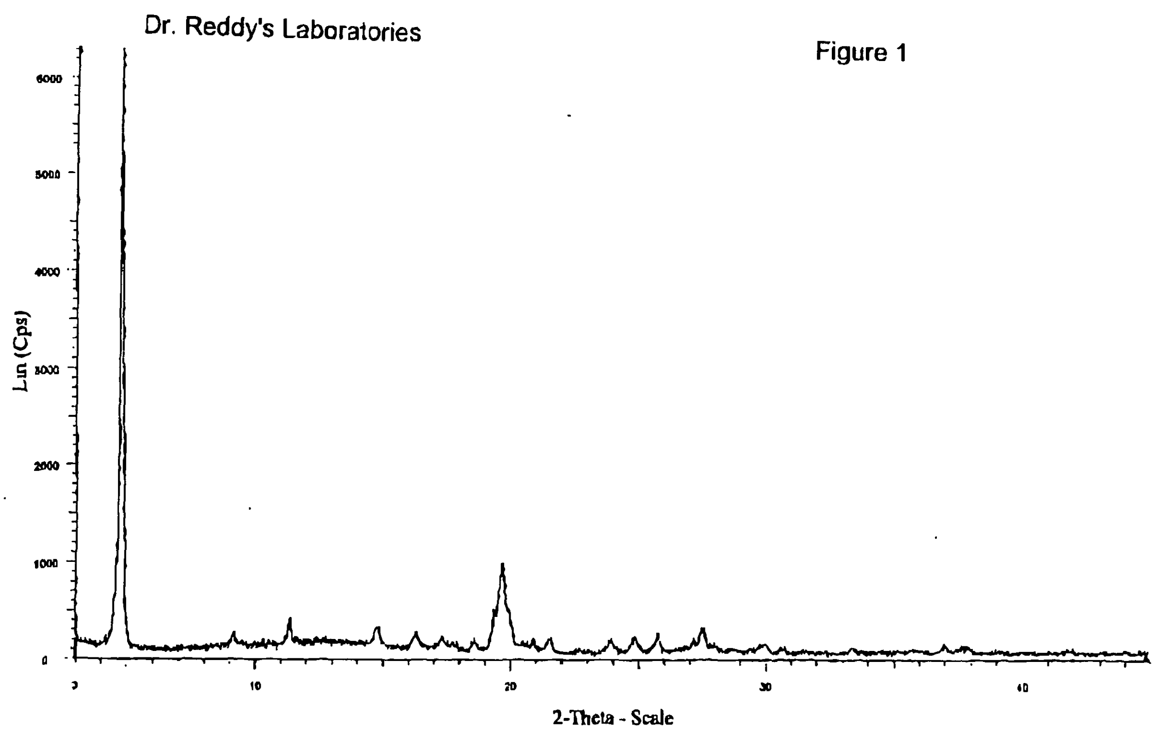


Figure 1



Printed by Jouve, 75001 PARIS (FR)

## Description

CROSS-REFERENCE TO RELATED APPLICATION

This application claims priority of Indian Patent Application No. 156/MAS/2003 filed on February 28, 2003, the disclosure of which is incorporated herein by reference in its entirety.

BACKGROUND OF THE INVENTION

Rabeprazole sodium, 2-[[[4-(3-methoxypropoxy)-3-methyl-2-pyridinyl]-methyl]sulfonyl]-1*H*-benzimidazole sodium is an inhibitor of the gastric proton pump. It belongs to a class of antisecretory compounds that do not exhibit anticholinergic or histamine $H_2$-receptor antagonist properties, but suppress gastric acid secretion by inhibiting the gastric $H^+$, $K^+$ ATPase at the secretory surface of the gastric parietal cell. Rabeprazole blocks the final step of gastric acid secretion.

SUMMARY OF THE INVENTION

In accordance with one aspect, the invention provides a new crystalline Form Z of rabeprazole sodium. Preferably, crystalline Form Z of rabeprazole sodium has an X-ray diffraction pattern, expressed in terms of 2 theta angles, that includes four or more peaks selected from the group consisting of 4.69 ±0.09, 9.07±0.09, 9.42±0.09, 11.25±0.09, 14.71±0.09, 16.24±0.09, 17.26±0.09, 18.52±0.09, 18.52±0.09, 19.32±0.09, 19.63±0.09, 19.92±-0-09, 20.80±0.09, 21.48±0.09, 23.07±0.09, 24.81±0.09, 25.70±0.09, 27.47±0.09, 30.01±0.09, 30.65±0.09, 33.37±0.09, and 36.95±0.09. Various embodiments and variants are also provided.

In accordance with another aspect, the invention provides a composition that includes rabeprazole sodium in a solid form, wherein at least 80 % by weight of the solid rabeprazole sodium is the crystalline Form Z of rabeprazole sodium. Various embodiments and variants are also provided.

In accordance with yet other aspects, the invention provides a process for preparing the crystalline Form Z of rabeprazole sodium, and a pharmaceutical composition that includes the crystalline Form Z of rabeprazole sodium and one or more pharmaceutically acceptable carriers or diluents. The pharmaceutical composition may also include one or more additional active ingredients. Preferably, the pharmaceutical composition is in a solid dosage form for oral administration, such as a tablet.

The invention also relates to a method of preventing or treating a disease that is associated with excess gastric acid secretion, comprising administering to a patient in need of such treatment an effective amount of crystalline Form Z of rabeprazole sodium.

BRIEF DESCRIPTION OF THE ACCOMPANYING DRAWINGS

Figure 1 shows an example of an X-ray power diffractogram of the crystalline Form Z of rabeprazole sodium.

Figure 2 shows a example of a differential scanning calorimetry thermogram of the crystalline Form Z of ziprasidone sodium.

DETAILED DESCRIPTION OF THE INVENTION

Unless defined otherwise, all technical and scientific terms used herein have the same meaning as commonly understood by one of ordinary skill in the art, to which this invention belongs. Although any methods and materials similar or equivalent to those described herein can be used in the practice or testing of the present invention, the preferred methods and materials are described.

Unless stated to the contrary, any use of the words such as "including," "containing," "comprising," "having" and the like, means "including without limitation" and shall not be construed to limit any general statement that it follows to the specific or similar items or matters immediately following it. Embodiments of the invention are not mutually exclusive, but may be implemented in various combinations. The described embodiments of the invention and the disclosed examples are given for the purpose of illustration rather than limitation of the invention as set forth the appended claims.

For purposes of the present invention, the following terms are defined below.

A "compound" is a chemical substance that includes molecules of the same chemical structure.

"Pharmaceutically acceptable" means that which is useful in preparing a pharmaceutical composition that is generally non-toxic and is not biologically undesirable and includes that which is acceptable for veterinary use and/or human pharmaceutical use.

The term "composition" includes, but is not limited to, a powder, a suspension, an emulsion and/or mixtures thereof. The term composition is intended to encompass a product containing the specified ingredients in the specified amounts, as well as any product, which results, directly or indirectly, from combination of the specified ingredients in the specified amounts. A "composition" may contain a single compound or a mixture of compounds.

The term "pharmaceutical composition" is intended to encompass a product comprising the active ingredient(s), pharmaceutically acceptable excipients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the active ingredient, additional active ingredient(s), and pharmaceutically acceptable excipients.

The term "excipient" means a component of a pharmaceutical product that is not the active ingredient, such as filler, diluent, carrier, and so on. The excipients that are useful in preparing a pharmaceutical composition are preferably generally safe, non-toxic and neither biologically nor otherwise undesirable, and are acceptable for veterinary use as well as human pharmaceutical use. "A pharmaceutically acceptable excipient" as used in the specification and claims includes both one and more than one such excipient.

When referring to a chemical reaction, the terms "treating", "contacting" and "reacting" are used interchangeably herein and refer to adding or mixing two or more reagents under appropriate conditions to produce the indicated and/or the desired product. It should be appreciated that the reaction, which produces the indicated and/or the desired product, may not necessarily result directly from the combination of two reagents, which were initially added, *i.e.,* there may be one or more intermediates which are produced in the mixture which ultimately leads to the formation of the indicated and/or the desired product. Also, the term "isolating" is used to indicate separation of the compound being isolated regardless of the purity of the isolated compound from any unwanted substance which presents with the compound as a mixture. Thus, degree of the purity of the isolated or separated compound does not affect the status of "isolating".

The term "substantially free of" in reference to a composition, as used herein, means that said substance cannot be detected in the composition by methods known to those skilled in the art at the time of the filing of this application.

Rabeprazole sodium has the chemical structure:

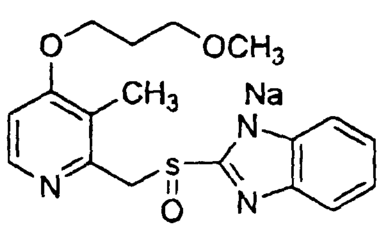

The preparation of rabeprazole is known in the art. For example, processes for the preparation of rabeprazole and its sodium salt are disclosed in U.S. Patent No. 5,045,552, the entire content of which is incorporated herein by reference. The disclosed process for preparing rabeprazole involves oxidation of 2-[{4-(3-methoxypropoxy)-3-methylpyridine-2-yl}methylthio]-1H-benzimidazole with m-chloroperbenzoic acid to afford rabeprazole base. The base is then converted to rabeprazole sodium salt in aqueous sodium hydroxide solution. Rabeprazole sodium prepared by the process of U.S. Patent No. 5,045,552 is characterized as a white crystal, which is precipitated from ether solvent. The melting point of the disclosed rabeprazole sodium is 140-141°C.

Three different types of crystalline forms of rabeprazole sodium have been previously reported. One is disclosed in Japanese patent 2001-39975, wherein the disclosed crystalline form is designated as Crystal II. The X-ray diffractogram peaks for Crystal II is also disclosed and reproduced in the following Table 1:

Table 1

| Form II | |
|---|---|
| 2θ(°) | Intensity ($I/I_0$) |
| 19.52 | 100 |
| 17.20 | 41 |
| 26.60 | 28 |
| 20.92 | 18 |
| 18.04 | 17 |
| 24.76 | 13 |
| 21.20 | 12 |
| 14.22 | 10 |
| 17.60 | 10 |
| 25.00 | 10 |
| 29.40 | 10 |
| 28.76 | 9 |
| 27.56 | 7 |
| 27.76 | 7 |
| 12.54 | 5 |
| 13.20 | 5 |
| 24.38 | 5 |
| 28.50 | 5 |
| 34.04 | 5 |
| 13.80 | 4 |
| 22.64 | 4 |
| 24.16 | 4 |
| 30.00 | 4 |
| 12.82 | 3 |
| 31.62 | 3 |
| 11.84 | 2 |

Table 1 (continued)

| Form II | |
|---|---|
| 2θ(°) | Intensity ($I/I_0$) |
| 25.92 | 2 |
| 34.92 | 2 |
| 8.88 | 1 |
| 9.64 | 1 |

The process for preparing Crystal II as disclosed in the Japanese patent includes crystallization of amorphous rabeprazole sodium or acetone complex of rabeprazole sodium in ethyl acetate, isopropyl acetate, isobutyl acetate, ethyl propionate, isobutyl propionate or ethyl butyrate.

In addition to Crystal II form of rabeprazole sodium, two other crystalline forms of rabeprazole sodium were previously disclosed in WO 03/082858 A1, incorporated herein by reference, wherein the two crystalline forms of rabeprazole sodium were designated as Forms X and Y respectively. The X-ray diffractograms peaks of the Forms X and Y are shown in Table 2:

Table 2

| Form X | | Form Y | |
|---|---|---|---|
| 2θ(°) | Intensity (cps) | 2θ(°) | Intensity (cps) |
| 5.13 | 1184 | 5.61 | 1635 |
| 6.606 | 225 | 19.442 | 546 |
| 20.01 | 215 | 18.816 | 329 |
| 23.469 | 193 | 7.725 | 285 |
| 8.569 | 169 | 7.207 | 242 |
| 12.923 | 154 | 9.646 | 235 |
| 20.539 | 135 | 10.352 | 219 |
| 22.177 | 131 | 16.899 | 186 |
| 24.81 | 125 | 24.943 | 162 |
| 10.565 | 125 | 16.418 | 104 |
| 12.161 | 116 | 14.546 | 103 |
| 9.353 | 113 | 11.231 | 77 |
| 18.173 | 99.3 | | |
| 17.309 | 85.3 | | |
| 14.864 | 81.3 | | |
| 24.494 | 75.3 | | |
| 16.372 | 69.9 | | |
| 14.414 | 60.2 | | |
| 7.244 | 57.5 | | |

Table 2 (continued)

| Form X | | Form Y | |
|---|---|---|---|
| 2θ(°) | Intensity (cps) | 2θ(°) | Intensity (cps) |
| 19.072 | 56.2 | | |

The differential scanning calorimetry thermogram of crystalline Form X exhibits a significant endo-exo pattern at 154.62°C and 214.65°C and that of crystalline Form Y shows peaks at 182.61°C and 215.57°C. The melting points of the Forms X and Y were also measured by the capillary method respectively as 140-150°C and 160-170°C.

It is known that polymorphic forms of the same drug may have substantial differences in certain pharmaceutically important properties such as dissolution characteristics and bioavailability as well as stability of the drug. Furthermore, difference crystalline forms may have different particle size, hardness and glass transition temperature. Thus, one crystalline form may provide significant advantages over other crystalline forms of the same drug in solid dosage form manufacture process such as accurate measurement of the active ingredients, easier filtration, or improved stability, including light and thermal stability, during granulation or storage. Furthermore, a particular process suitable for one crystalline form may also provide drug manufacturers several advantages such as economically or environmentally suitable solvents or process, or higher purity or yield of the desired product.

Thus, according to one aspect, the present invention provides a novel crystalline form of rabeprazole sodium, which is different from the Crystal II, the Form X or the Form Y. The specific crystalline form obtained by the inventors is designated as Form Z. Crystalline Form Z may be prepared directly from unpurified rabeprazole sodium or purified amorphous or other crystalline forms of rabeprazole sodium. For example, rabeprazole sodium may be prepared by reacting rabeprazole with sodium hydroxide in methanol. Rabeprazole sodium prepared by the reaction can be used for the preparation process of crystalline Form Z without vigorous purification steps. Also, amorphous form or other crystalline forms of rabeprazole sodium such as Form II, X or Y can be conveniently converted to crystalline Form Z by the process described below. Regardless of whether unpurified rabeprazole sodium or amorphous or other crystalline forms of rabeprazole sodium is used as the starting material, a general process for crystalline Form Z may involve providing rabeprazole sodium, which is not crystalline Form Z, in an aromatic hydrocarbon solvents; refluxing the reaction mixture azeotropically; cooling the refluxed reaction mixture. The aromatic hydrocarbon solvent is preferably toluene, xylenes or mixtures thereof and may be used in an amount so that the amount ratio (w/v) between rabeprazole sodium and

the aromatic hydrocarbon solvent is preferably between about 1:3 and about 1:20, more preferably between about 1:3 and about 1:10, even more preferably 1:4. The reflux duration can vary depending upon the particular amount ratio or the total amount of rabeprazole and the solvents adopted for the process. Preferably, the reflux is continued for about 1-10 hours, more preferably for about 2-8, even more preferably about 2-6.

Conventional methods for isolating solids from liquid media such as filtration can be used to isolate crystalline Form Z of rabeprazole sodium prepared by the above process.

The crystalline Form Z of rabeprazole sodium produced by this process was characterized by an X-ray powder diffraction pattern, as for example shown in Figure 1, and the characteristic 2 theta values (in degrees) and intensities of the identified peaks in the X-ray diffractograms are shown in Table 3:

Table 3

| Form Z | | |
|---|---|---|
| 2θ(°) | Intensity (cps) | Intensity ($I/I_0$) |
| 4.694 | 5710 | 100 |
| 19.320 | 829 | 14.5 |
| 19.626 | 368 | 6.4 |
| 18.522 | 297 | 5.2 |
| 11.254 | 200 | 3.5 |
| 14.712 | 181 | 3.2 |
| 27.470 | 179 | 3.1 |
| 9.070 | 147 | 2.6 |
| 16.241 | 141 | 2.5 |
| 24.814 | 143 | 2.5 |
| 25.702 | 138 | 2.4 |
| 20.802 | 122 | 2.1 |
| 23.073 | 110 | 1.9 |
| 17.264 | 94 | 1.6 |
| 18.522 | 87.9 | 1.5 |
| 19.920 | 84.6 | 1.5 |
| 36.950 | 61 | 1.1 |
| 30.009 | 56.5 | 1.0 |
| 33.365 | 49 | 0.9 |
| 9.417 | 42.1 | 0.7 |
| 21.477 | 40.2 | 0.7 |
| 30.653 | 39 | 0.7 |

The X-ray diffractogram was measured on Bruker Axe, DS advance Power X-ray Diffractometer with Cu K alpha-1 Radiation source.

It should be kept in mind that slight variations in the observed 2 theta angles values are expected based on the specific diffractometer employed, the analyst and the sample preparation technique. More variation is expected for the relative peak intensities. Identification of the exact crystalline form of a compound should be based primarily on observed 2 theta angles with lesser importance attributed to relative peak intensities. The peaks reported herein are listed in order of their peak intensities. Thus, the first listed peak has stronger intensity than the second listed peak in the pattern. The 2 theta diffraction angles and corresponding d-spacing values account for positions of various peaks in the X-ray powder diffraction pattern. D-spacing values are calculated with observed 2 theta angles and copper K(α1) wavelength using the Bragg equation well known to those of skill in the art.

Thus, some margin of error may be present in each of the 2 theta angle assignments reported herein. The assigned margin of error in the 2 theta angles for the crystalline Form Z of rabeprazole is approximately ± 0.009 for each of the peak assignments. In view of the assigned margin of error, in a preferred variant, the crystalline form of rabeprazole may be characterized by an X-ray diffraction pattern, expressed in terms of 2 theta angles, that includes four or more peaks selected from the group consisting of 4.69 ±0.09, 9.07±0.09, 9.42±0.09, 11.25±0.09, 14.71±0.09, 16.24±0.09, 17.26±0.09, 18.52±0.09, 18,52±0.09, 19.32±0.09, 19.63±0.09, 19.92±0.09, 20.80±0.09, 21.48±0.09, 23.07±0.09, 24.81±0.09, 25.70±0.09, 27.47±0.09, 30.01±0.09, 30.65±0.09, 33.37±0.09, and 36.95±0.09.

Since some margin of error is possible in the assignment of 2 theta angles and d-spacings, the preferred method of comparing X-ray powder diffraction patterns in order to identify a particular crystalline form is to overlay the X-ray powder diffraction pattern of the unknown form over the X-ray powder diffraction pattern of a known form. For example, one skilled in the art can overlay an X-ray powder diffraction pattern of an unidentified crystalline form of rabeprazole obtained using the methods described herein, over FIG. 1 and readily determine whether the X-ray diffraction pattern of the unidentified form is substantially the same as the X-ray powder diffraction pattern of the crystalline form of this invention. If the X-ray powder diffraction pattern is substantially the same as FIG. 1, the previously unknown crystalline form of rabeprazole can be readily and accurately identified as the crystalline form of this invention.

The crystalline forms of rabeprazole sodium may also be characterized by differential scanning calorimetry (DSC). The DSC thermogram of crystalline Form Z of rabeprazole sodium obtained by the inventors is shown in Figure 2. It exhibits a significant endo-exo pattern with identified peaks around 106.5 °C and 228.8 °C. The melting point of the crystalline Form Z was also measured by the capillary method and was determined

to be 224-230°C.

The invention also relates to a composition containing solid rabeprazole sodium of which at least 80%, by total weight of the solid rabeprazole sodium in the composition, is crystalline Form Z. In the more preferred form of this composition, the solid rabeprazole sodium is suitable for use as active ingredient in formulating pharmaceutical products. The remainder of the solid rabeprazole sodium in the composition, *i.e.,* 20% or less of the total weight of rabeprazole sodium, may be amorphous form or crystalline Form II, X or Y of rabeprazole sodium or mixtures thereof In an embodiment of the invention, the composition may comprise at least 90% of crystalline Form Z of rabeprazole sodium with respect to total weight of the solid rabeprazole sodium in the composition. In another embodiment of the invention, the composition may comprise at least 95% of crystalline Form Z of rabeprazole sodium with respect to total weight of the solid rabeprazole sodium in the composition. In yet another embodiment of the invention, the composition is substantially free of any forms of rabeprazole sodium other than its crystalline Form Z.

X-ray diffraction provides a convenient and practical means for quantitative determination of the relative amounts of crystalline and/or amorphous forms in a solid mixture. X-ray diffraction is adaptable to quantitative applications because the intensities of the diffraction peaks of a given compound in a mixture are proportional to the fraction of the corresponding powder in the mixture. The percent composition of crystalline rabeprazole sodium in an unknown composition can be determined. Preferably, the measurements are made on solid powder rabeprazole sodium. The X-ray powder diffraction patterns of an unknown composition can be compared to known quantitative standards containing pure crystalline forms of rabeprazole sodium to identify the percent ratio of a particular crystalline form. This is done by comparing the relative intensities of the peaks from the diffraction pattern of the unknown solid powder composition with a calibration curve derived from the X-ray diffraction patterns of pure known samples. The curve can be calibrated based on the X-ray powder diffraction pattern for the strongest peak from a pure sample of crystalline forms of rabeprazole sodium. The calibration curve may be created in a manner known to those of skill in the art. For example, five or more artificial mixtures of crystalline forms of rabeprazole sodium, at different amounts, may be prepared. In a non-limiting example, such mixtures may contain, 2%, 5%, 7%, 8%, and 10% of rabeprazole sodium for each crystalline form. Then, X-ray diffraction patterns are obtained for each artificial mixture using standard X-ray diffraction techniques. Slight variations in peak positions, if any, may be accounted for by adjusting the location of the peak to be measured. The intensities of the selected characteristic peak(s) for each of the artificial mixtures are then plotted against the known weight percentages of the crystalline form. The resulting plot is a calibration curve that allows determination of the amount of the crystalline forms of rabeprazole sodium in an unknown sample. For the unknown mixture of crystalline and amorphous forms of rabeprazole sodium, the intensities of the selected characteristic peak(s) in the mixture, relative to an intensity of this peak in a calibration mixture, may be used to determine the percentage of the given crystalline form in the composition, with the remainder determined to be the amorphous material.

Pharmaceutical compositions comprising crystalline Form Z of rabeprazole can be formulated with one or more pharmaceutically acceptable carriers, also known as excipients, which ordinarily lack pharmaceutical activity, but have various useful properties which may, for example, enhance the stability, sterility, bioavailability, and ease of formulation of a pharmaceutical composition. These carriers are pharmaceutically acceptable, meaning that they are not harmful to humans or animals when taken appropriately and are compatible with the other ingredients in a given formulation. The carriers may be solid, semi-solid, or liquid, and may be formulated with the compound in bulk. The resulting mixture may be manufactured in the form of a unit-dose formulation (i.e., a physically discrete unit containing a specific amount of active ingredient) such as a tablet or capsule.

The pharmaceutical compositions may include, in addition to a compound of this invention, one or more active pharmaceutical compounds in order to achieve synergistic effects. For example, U.S. Patent 6,303,644, incorporated by reference, discloses use of a proton pump inhibitors as combination with an antimicrobial compound to avoid or reduce side effects caused by the antimicrobial compound. Similarly, U.S. Patent 6,117,868, incorporated by reference, discloses treatment of infectious gastrointestinal ulcer disease or infectious gastritis disease by administering an antimicrobial medicament in combination with a proton pump inhibitor. Thus, the additional pharmaceutical compound to be added to the pharmaceutical composition of the present invention may includes, for example, antibacterial or antimicrobial compounds such as penicillins including benzylpenicillin, phenoxymethylpenicillin, propicillin, azidicillin, dicioxacillin, flucloxacillin, oxacillin, amoxicillin, bacampicillin, ampicillin, meziocillin, piperacillin, or aziocillin; cephalosporins including cefadroxil, cefaclor, cefalexin, cefalexim, cefuroxim, cefetamet, cefadroxil, ceftibuten, cefpodoxim, cefotetan, cefazolin, cefoperazon, ceftizoxim, ceftaxim, ceftazidim, cefamandol, cefepim, cefoxitin, cefodizim, cefsulodin, ceftriaxon, cefotiam, or cefmenoxim; aztreonam; loracarbef; meropencm; sulbactam; tetracyclines including tetracycline, oxytetracycline, minocycline, or doxycycline; aminoglycosides including tobramycin, gentamicin, neomycin, streptomycin, amikacin, netilmicin, paromomycin or spectinomycin; ampherlcols including chloramphenicol or thiamphenicol; lincomycins; clindamycin; lincomycin; erythromycin; clarithromycin; spiramycin; roxithromycin; azithromycin; collstin; polymixin B; teioplanin; vancomycin; norfloxacin; cinoxacin; ciprofloxacin; pipemidic acid; enoxacin; nalidixie acid; pefloxacin; fieroxacin; ofloxacin; metronidazole; fomycin; fucidic acid; taurolidine; taurultam; and mixtures thereof.

Generally, the pharmaceutical compositions of the invention may be prepared by uniformly admixing the active ingredient with liquid or solid carriers and then shaping the product into the desired form. The pharmaceutical compositions may be in the form of suspensions, solutions, elixirs, aerosols, or solid dosage forms. Because of their ease of administration, tablets and capsules represent the most advantageous oral dosage unit form, in which case solid pharmaceutical carriers are employed.

A preferred oral solid preparation is a tablet. A tablet may be prepared by direct compression, wet granulation, or molding, of the active ingredient(s) with a carrier and other excipients in a manner known to those skilled in the art. Compressed tablets may be prepared by compressing in a suitable machine the active ingredient in a free-flowing form such as powder or granules, optionally mixed with a binder, lubricant, inert diluent, surface active agent or dispersing agent. Molded tablets may be made on a suitable machine. A mixture of the powdered compound moistened with an inert liquid diluent is suitable in the case of oral solid dosage forms (e. g., powders, capsules, and tablets). If desired, tablets may be coated by standard techniques. The compounds of this invention may be formulated into typical disintegrating tablets, or into controlled or extended release dosage forms.

The pharmaceutical compositions of the invention are contemplated in various formulations suitable for various modes of administration, including but not limited to inhalation, oral, rectal, parenteral (including subcutaneous, intradermal, intramuscular, intravenous), implantable, intravaginal and transdermal administration. The most suitable route of administration in any given case depends on the duration of the subject's condition, the length of treatment desired, the nature and severity of the condition being treated, and the particular formulation that is being used. The formulations may be in bulk or in unit dosage form.

The amount of active ingredient included in a unit dosage form depends on the type of formulation that is formulated. A pharmaceutical composition of the invention will generally comprise about 0.1% by weight to about 99% by weight of active ingredient, preferably about 1% by weight to 50% by weight for oral administration and about 0.2% by weight to about 20% by weight for parenteral administration.

Formulations suitable for oral administration include capsules (hard and soft), cachets, lozenges, syrups, suppositories, and tablets, each containing a pre-determined amount of the active compound; as a powder or granules; as a solution or a suspension in an aqueous or non-aqueous liquid; or as an oil-in-water or water-in-oil emulsion. Such formulations may be prepared by any suitable method of pharmacy that includes the step of bringing into association the active compound and a suitable carrier or carriers. The amount of active ingredient per unit dosage of solid formulations may be as described in prior art for preparations of rabeprazole sodium. For liquid oral formulations, a preferable amount is from about 2% by weight to about 20% by weight. Suitable carriers include but are not limited to fillers, binders, lubricants, inert diluents, surface active/dispersing agents, flavorants, antioxidants, bulking and granulating agents, adsorbants, preservatives, emulsifiers, suspending and wetting agents, glidants, disintegrants, buffers and pH-adjusting agents, and colorants. Examples of carriers include celluloses, modified celluloses, cyclodextrins, starches, oils, polyols, sugar alcohols and sugars, and others. For liquid formulations sugar, sugar alcohols, ethanol, water, glycerol, and poyalkylene glycols are particularly suitable, and may also be used in solid formulations. Cyclodextrins may be particularly useful for increasing bioavailability. Formulations for oral administration may optionally include enteric coatings known in the art to prevent degradation of the formulation in the stomach and provide release of the drug in the small intestine.

Formulations suitable for buccal or sub-lingual administration include lozenges comprising the active compound in a flavored base, usually sucrose and acacia or tragacanth, although other agents are also suitable, and pastilles comprising the compound in an inert base such as gelatin and glycerin or sucrose and acacia.

Formulations suitable for rectal administration are preferably presented as unit dose suppositories. These may be prepared by admixing the active compound with one or more conventional solid carriers, e. g., cocoa butter, and then shaping the resulting mixture.

In another aspect, the invention also provides methods of preventing or treating a disease that is associated with excess gastric acid secretion, comprising administering to a patient in need of such treatment an effective amount of crystalline Form Z of rabeprazole sodium. In particular, for example, the method of the present invention can be used in healing or maintenance of erosive or ulcerative Gastroesophageal Reflux Disease (GERD), treatment of symptomatic Gastroesophageal Reflux Disease, healing of Duodenal Ulcers, helicobacter pylori eradication to reduce the risk of Duodenal Ulcer recurrence, and treatment of pathological hypersecretory conditions, including Zollinger-Ellison Syndrome.

The effective amount (i.e., dosage) of active compound for treatment will vary depending on the route of administration, the condition being treated, its severity, and duration, and the state and age of the subject. A skilled physician will monitor the progress of the subject and will adjust the dosage accordingly, depending on whether the goal is to eliminate, alleviate, or prevent a given condition. Generally, the dosage should be considered in proportion to the subject's weight. The daily dose of particular formulations of active compound may be divided among one or several unit dose administrations. For example therapeutic administration about fifteen to thirty minutes before main meals is preferable (i. e. three times daily), although administration of the active compounds may be carried out prophylactically, and may be maintained for prolonged periods of time. One skilled in the art will take such factors into account when determining dosage. Unit dosage of active ingredient may range preferably from about 1mg to about 100 mg, more preferably from about 10 mg to about 50 mg.

The invention is further described by reference to the following examples which set forth in detail the preparation of compounds and compositions of the present invention, as well as their utility. It will be apparent to those skilled in the art, that many modifications, both to materials, and methods, may be practiced without departing from the purpose and interest of this invention. The examples that follow are not intended to limit the scope of the invention as described hereinabove or as claimed below.

Reference Example 1:

Preparation of 2-[[[4-(3-methoxypropoxy)-3-methyl-2-pyridinyl]-methyl] sulfinyl]-1*H*-benzimidazole (Rabeprazole)

2-[[4-(3-methoxypropoxy)-3-methyl-2-pyridinyl]-methyl thio]-1*H*-benzimidazole (Prepared as per example 90 of the Patent No. 5,045,552) (100 grams, 0.29 moles) was added to a mixture of chloroform (9500 ml) and dimethylsulphoxide (200 ml) and the reaction mixture was cooled to -10°C to -15°C. 3-Chloroperbenzoic acid (60 grams, 0.24 moles) was dissolved in chloroform (500ml), and the chloroform solution was then added to the above solution at -10°C to -15°C over about 1- 2 hours, and the reaction mixture was maintained at the same temperature for 30 minutes. Then, 12.8 % (w/v) aqueous sodium hydroxide solution (500 ml) was added to the reaction mixture. The pH of the reaction mixture was adjusted to 9.5 to 10.0 with acetic acid, which formed a biphasic system. The organic layer was separated and then extracted with 1.6 % (w/v) aqueous sodium hydroxide solution (500 ml). The sodium hydroxide extract was diluted with a mixture of chloroform (140 ml) and methanol (100 ml). Then the pH of the mass was again adjusted to 9.5 to 10.0 with acetic acid, and the organic layer was separated again. To the separated organic layer was added tert. butyl methyl ether (440 ml). The reaction mixture was stirred for about 1-2 hours at a temperature of 0-5 °C and was subjected to filtration. The residue was dissolved in a mixture of 110 % (w/v) aqueous sodium hydroxide solution (100 ml) and methanol (65 ml). The pH of the reaction solution was adjusted to 9.0 to 9.5 with acetic acid at 10-15 °C and was stirred for additional 2 hours, followed by filtration. The wet material is then dissolved in dichloromethane (130 ml), and the water layer separated where after solution was added to tert. butyl methyl ether (260 ml), stirred at a temperature of 0-5°C for 1-2 hours. The precipitated solid was filtered and dried to give 2-[[[4-(3-methoxypropoxy)-3-methyl-2-pyridinyl]-methyl] sulfinyl]-1*H*-benzimidazole.

Reference Example 2

Preparatjon of amorphous form of 2-[[[4-(3-methoxypropoxy)-3-methyl-2-pyridinyl]-methyl]sulfinyl]-1*H*-benzimidazole sodium (rabeprazole sodium)

2-[[[4-(3-methoxypropoxy)-3-methyl-2-pyridinyl]-methyl]sulfinyl]-1*H*-benzimidazole (obtained as per reference example 1) (50.0 grams, 0.139 moles) was dissolved in a mixture of sodium hydroxide (7.5 grams, 0.187 moles) and methanol (100.0 ml), and the solution was stirred at ambient temperature 25-35 °C. The reaction solution is filtered through hi-flow and washed with methanol (50.0 ml). Then, the solvent of the filtrate was distilled off under reduced pressure. The reaction mass was cooled to ambient temperature, and petroleum ether (400.0 ml) was then added to the residual mass, which was then stirred at 25-30 °C for about 1-2 hours. The precipitated solid was filtered and washed with petroleum ether (100.0 ml) and dried at 50-60°C for 12 hours to afford the desired amorphous form of rabeprazole sodium (Weight: 50.4 grams, 94.9%).

Reference Example 3

Preparation of Crystalline form X of 2-[[[4-(3-methoxypropoxy)-3-methyl-2-pyridinyl]-methyl]sulfinyl]-1*H*-benzimidazole sodium (Rabeprazole sodium)

2-[[[4-(3-methoxypropoxy)-3-melhyl-2-pyridinyl]-methyl] sulfinyl]-1*H*-benzimidazole (obtained by reference example 1) (50.0 grams, 0.139 moles) was dissolved in a mixture of sodium hydroxide (7.5 grams, 0.187 moles) and methanol (100.0 ml) and stirred at ambient temperature 25-35°C. The reaction solution was filtered through hi-flow and washed with methanol (50-0 ml). Then the solvent of the filtrate was distilled off under reduced pressure. The reaction mass was cooled to ambient temperature. Dichloromethane (100.0 ml) was added to the reaction mixture, which was then distilled to remove traces of methanol. Dichloromethane (50.0 ml) and petroleum ether (100.0 ml) was then added to the residual mass, which was then stirred at 25-30 °C for about 6-8 hours. Additional petroleum ether (150 ml) was added to the reaction mixture, which was stirred at 25-30 °C for 1-2 hours. The precipitated solid was filtered and washed with petroleum ether (100.0 ml) and dried at 50-60°C for 12 hours to afford the desired form

X of rabeprazole sodium (Weight: 50.4 grams, 94.9%).

Reference example 4

Preparation of Crystalline form-Y of 2-[[[4-(3-methoxypropoxy)-3-methyl-2-pyridinyl]-methyl]sulfinyl]-1*H*-benzimidazole sodium (rabeprazole sodium)

2-[[[4-(3-methoxypropoxy)-3-methyl-2-pyridinyl]-methyl]sulfinyl]-1*H*-benzimidazole (obtained as per reference example 1) (750.0 grams, 2.089 moles) was dissolved in a mixture of sodium hydroxide (112.5 grams, 2.8125 moles) and methanol (1500.0 ml), and the resulting solution was stirred at ambient temperature 25-35°C. The reaction solution was filtered through hi-flow and washed with methanol (750.0 ml). The solvent of the filtrate was distilled off completely under reduced pressure. The reaction mass was cooled to ambient temperature, and dichloromethane (1500.0 ml) was added to the reaction mass. The solvent was distilled again to remove traces of methanol. The reaction mass was cooled to ambient temperature, and n-butanol (375.0 ml) and tertiary butyl methyl ether (6.0 L) was added to the residual mass which is stirred at 25-30°C for 6-8 hours. The reaction mixture was further cooled to 5-15 °C and then stirred for another 3-5 hours. The solid then precipitated was filtered and washed with tertiary butyl methyl ether (1500.0 ml) and dried at 50-60°C for 7 hours to afford the desired crystalline Form Y of rabeprazole sodium (Weight: 725.0 grams, 91.1%).

Example 1

Preparation of Crystalline form Z of 2-[[[4-(3-methoxypropoxy)-3-methyl-2-2-pyridinyl]-methyl]sulfinyl]-1*H*-benzimidazole sodium (Rabeprazole sodium) from 2-[[[4-(3-methoxypropoxy)-3-methyl-2-pyridinyl]-methyl]sulfinyl]-*1H*-benzimidazole (rabeprazole)

2-[[[4-(3-methoxypropoxy)-3-methyl-2-pyridinyl]-methyl]sulfinyl]-1*H*-benzimidazole (obtained by reference example 1) (50.0 grams, 0.139 moles) was dissolved in a mixture of sodium hydroxide (7.5 grams, 0.187 moles) and methanol (100.0 ml), and the resulted solution was stirred at ambient temperature 25-35°C for about one hour. The reaction solution was filtered through hi-flow and washed with methanol (50.0 ml). Then the solvent of the filtrate was distilled off under reduced pressure. The reaction mass is cooled to ambient temperature, to which toluene (200.0 ml) was added. The residual mass was then refluxed for about 2-6 hours. After cooling the reaction solution, the precipitated solid was filtered, washed with toluene (100.0 ml) and dried at 90-100°C for 12 hours to afford the desired form Z ofrabeprazole sodium (Weight: 50.4 grams, 94.9%).

Example 2

Preparation of Crystalline form 7 of 2-[[[4-(3-methoxypropoxy)-3-methyl-2-pyridinyl]-methyl]sulfinyl)-1*H*-benzimidazole sodium (rabeprazole sodium)

Crystalline Form X of rabeprazole Sodium (50.0 grams, 0.131 moles) was charged in toluene (200.0 ml) and the mixture was refluxed for 8-10 hours. The reaction mixture was then cooled to 25-35°C. The precipitated solid was filtered off, washed with toluene (100.0 ml) and dried at 90-100°C for 6-8 hours to afford the desired form Z of Rabeprazole sodium (Weight: 45 grams, 90%).

Example 3

Pharmaceutical composition of rabeprazole sodium crystalline Form Z

Rabeprazole sodium crystalline Form Z, precipitated calcium carbonate, corn starch, lactose, and hydroxypropylcellulose arc mixed together. Water is added to the mixture, which is then kneaded. The kneaded mixture is dried in vacuum at 40 °C for 16 hours, ground in a mortar and passed through a 16-mesh sieve to give granules. To this is added magnesium stearate, and the resultant mixture is made up into tablets.

| Composition per tablet | |
|---|---|
| Rabeprazole crystalline Form Z | 20 mg |
| Precipitated calcium carbonate | 50 mg |
| Corn Starch | 50 mg |
| Lactose | 73.4 mg |
| Hydroxypropylcellulose | 6 mg |
| Magnesium Stearate | (0.05 ml) |
| Total | 200.0 mg |

## Claims

1. A compound which is a crystalline Form Z of rabeprazole sodium.

2. The compound of claim 1 having an X-ray diffraction pattern, expressed in terms of 2 theta angles, that includes four or more peaks selected from the group consisting of 4.69±0.09, 9.07±0.09, 9.42±0.09, 11.25±0.09, 14.71±0.09, 16.24±0.09, 17.26±0.09, 18.52±0.09, 18.52±0.09, 19.32±0.09, 19.63±0.09, 19.92±0.09, 20.80±0.09, 21.48±0.09, 23.07±0.09, 24.81±0.09, 25.7±0.09, 27.47±0.09, 30.01±0.09, 30.65±0.09, 33.37±0.09, and 36.95±0.09.

3. The compound of claim 2 having an X-ray diffraction

pattern expressed in terms of 2 theta angles and obtained with a diffractometer equipped with a Cu K alpha-1 radiation source, wherein said X-ray powder diffraction pattern includes five or more peaks selected from the group consisting of peaks with 2 theta angles of about 4.694, 9.070, 9.417, 11.254, 14.712, 16-241, 17.264, 18.522, 18.522, 19.320, 19.626, 19.920, 20.802, 21.477, 23.073, 24.814, 25.702, 27.470, 30.009, 30.653, 33.365, and 36.950.

**4.** The compound of claim 1, having substantially the same X-ray diffraction pattern as shown in Figure 1.

**5.** The compound of claim 1, which has an endo-exo pattern with identified peaks of about 106.5 °C and 228.8 °C in its differential scanning calorimetry thermogram.

**6.** A composition comprising rabeprazole sodium as a solid, wherein at least 80 % by weight of said sold rabeprazole sodium is a crystalline Form Z of rabeprazole sodium.

**7.** The composition of claim 6, wherein at least 90 % by weight of said solid rabeprazole sodium is the crystalline Form Z.

**8.** The composition of claim 6, wherein at least 95 % by weight of said solid rabeprazole sodium is the crystalline Form Z.

**9.** The composition of claim 6, wherein at least 99 % by weight of said solid rabeprazole sodium is the crystalline Form Z.

**10.** The composition of claim 6, wherein said rabeprazole sodium is substantially free of amorphous form, Crystal II, Form X and Form Y of rabeprazole sodium.

**11.** A pharmaceutical composition comprising a prophylactically or therapeutically effective amount of the compound of claim 1 and one or more pharmaceutically acceptable excipients.

**12.** The pharmaceutical composition of claim 11, wherein said composition is a solid dosage form for oral administration.

**13.** The pharmaceutical composition of claim 11, wherein said dosage form is a tablet.

**14.** A pharmaceutical composition comprising a prophylactically or therapeutically effective amount of the compound of claim 1, one or more pharmaceutically acceptable excipients, and one or more antimicrobial compounds.

**15.** The pharmaceutical composition of claim 14, wherein said antimicrobial compound is selected from a group consisting of penicillins including benzylpenicillin, phenoxymethylpenicillin, propicillin, azidicillin, dicioxacillin, flucloxacillin, oxacillin, amoxicillin, bacampicillin, ampicillin, meziocillin, piperacillin, or aziocillin; cephalosporins including cefadroxil, cefaclor, cefalexin, cefalcxim, cefuroxim, cefetamet, cefadroxil, ceftibuten, cefpodoxim, cefotetan, cefazolin, cefoperazon, ceftizoxim, ceftaxim, ceftazidim, cefamandol, cefepim, cefoxitin, cefodizim, cefsulodin, ceftriaxon, cefotiam, or cefmenoxim; aztreonam; loracarbet; meropenem; sulbactam; tetracyclines including tetracycline, oxytetracycline, minocycline, or doxycycline; aminoglycosides including tobramycin, gentamicin, neomycin, streptomycin, amikacin, netilmicin, paromomycin or spectinomycin; ampherlcols including chloramphenicol or thiamphenicol; lincomycins; clindamycin; lincomycin; erythromycin; clarithromycin; spiramycin; roxithromycin; azithromycin; collstin; polymixin B; teioplanin; vancomycin; norfloxacin; cinoxacin; ciprofloxacin; pipemidic acid; enoxacin; nalidixie acid; pefloxacin; ficroxacin; ofloxacin; metronidazole; fomycin; fucidic acid; taurolidine; taurultam; and mixtures thereof.

**16.** A method of preventing or treating a disease that is associated with excess gastric acid secretion, comprising administering to a patient in need of said prevention or treatment an effective amount of the compound of claim 1.

**17.** The method of claim 16, wherein said disease is an ulcer, gastroesophageal reflux disease, psoriasis or Zollinger-Eliison Syndrome.

**18.** A process for making a crystalline Form Z of rabeprazole sodium, wherein said process comprising:

a. providing rabeprazole sodium in an aromatic hydrocarbon solvent;
b. heating said aromatic hydrocarbon solvent to reflux; and
c. cooling said solvent until a solid mass separates which is crystalline Form Z of rabeprazole sodium.

**19.** The process of claim 18, wherein said starting rabeprazole sodium is a crystalline form, an amorphous form or a mixture thereof.

**20.** The process of claim 18, wherein said aromatic hydrocarbon solvent is toluene, xylenes or mixtures thereof.

**21.** The process of claim 18, wherein said aromatic hydrocarbon solvent is toluene.

**22.** The process of claim 18, wherein said rabeprazole is provided in said aromatic hydrocarbon solvent in a ratio between about 1:3 and about 1:20.

**23.** The process of claim 22, wherein said ratio is between about 1:3 and about 1:10.

**24.** The process of claim 22, wherein said ratio is about 1:4.

**25.** A crystalline Form Z of rabeprazole sodium, which is prepared according to the process of claim 18.

Figure 1 of 2

Dr. Reddy's Laboratories

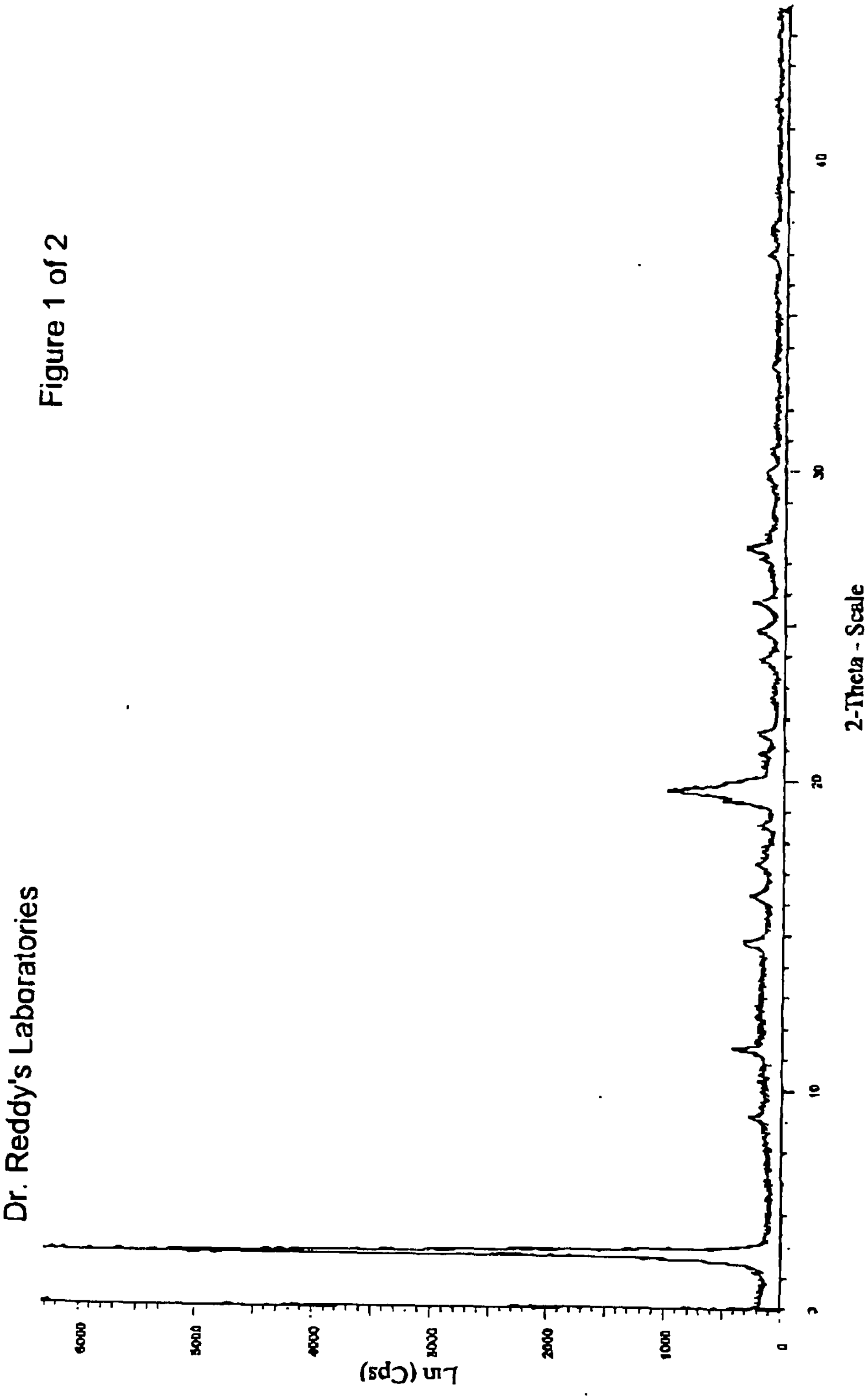

Dr. Reddy's Laboratories

Figure 2 of 2

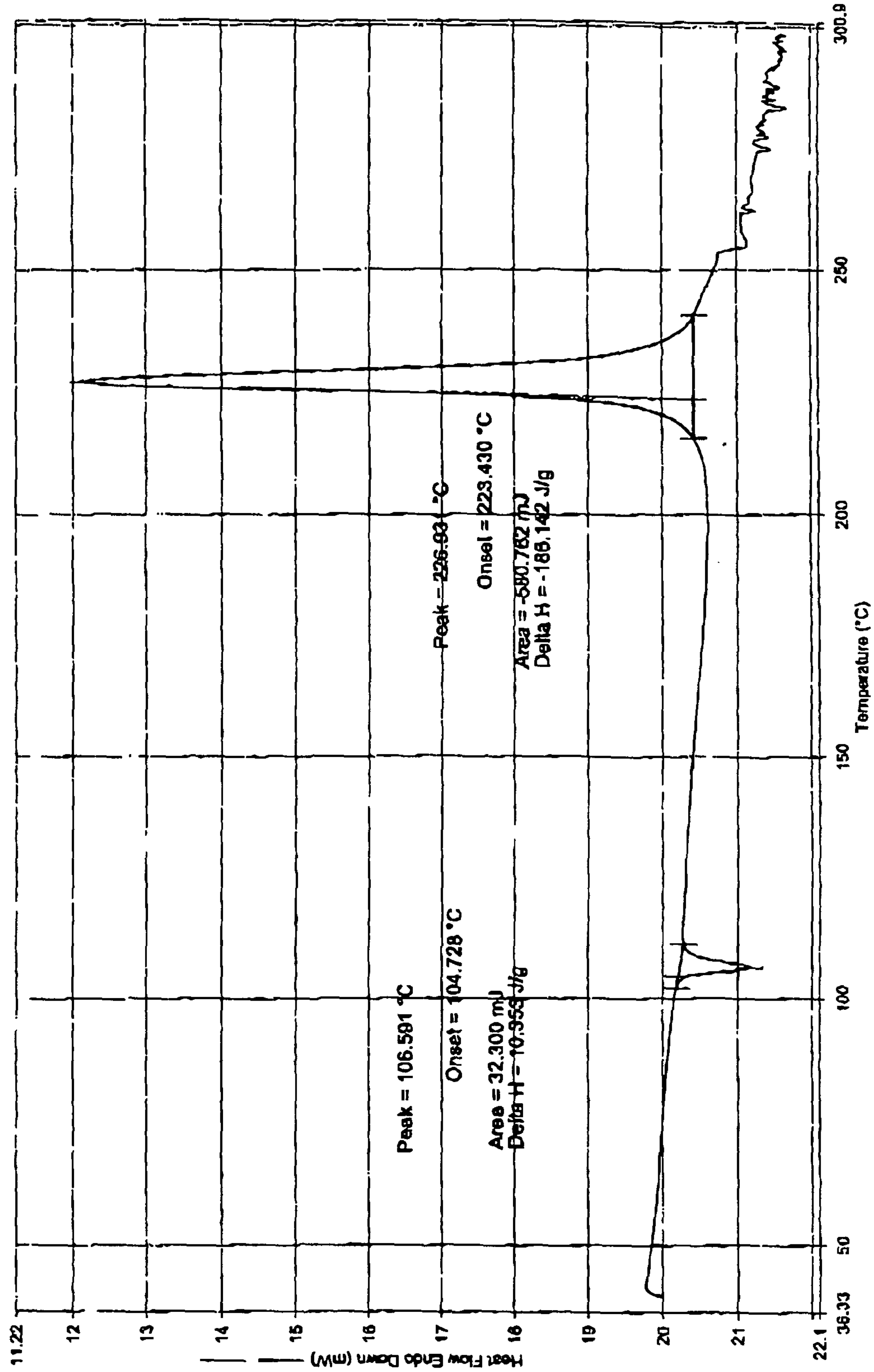

European Patent Office

# PARTIAL EUROPEAN SEARCH REPORT

which under Rule 45 of the European Patent Convention shall be considered, for the purposes of subsequent proceedings, as the European search report

Application Number EP 04 00 4420

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | PATENT ABSTRACTS OF JAPAN vol. 2000, no. 19, 5 June 2001 (2001-06-05) -& JP 2001 039975 A (EISAI CO LTD), 13 February 2001 (2001-02-13) * abstract * | 1-25 | C07D401/12<br>A61K31/4439<br>A61P1/04 |
| D,X | US 5 045 552 A (SOUDA S ET AL) 3 September 1991 (1991-09-03) * the whole document, particularly example 33 * | 1-25 | |
| A | WO 02/14286 A (TEVA PHARMACEUTICAL INDUSTRIES LTD.) 21 February 2002 (2002-02-21) * the whole document, particularly claim 23 * | 1-25 | |
| P,D,X | WO 03/082858 A (DR. REDDY'S LABORATORIES LIMITED) 9 October 2003 (2003-10-09) * the whole document * | 1-25 | |

TECHNICAL FIELDS SEARCHED (Int.Cl.7)

C07D
A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 26 May 2004 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 04 00 4420

Although claims 16 and 17 are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 04 00 4420

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2004

| Patent document cited in search report | Publication date | Patent family member(s) | Publication date |
|---|---|---|---|
| JP 2001039975 A | 13-02-2001 | NONE | |
| US 5045552 A | 03-09-1991 | AT 103912 T | 15-04-1994 |
| | | AT 163011 T | 15-02-1998 |
| | | AT 168111 T | 15-07-1998 |
| | | AU 594836 B2 | 15-03-1990 |
| | | AU 8113887 A | 19-05-1988 |
| | | CA 1265138 A1 | 30-01-1990 |
| | | CN 87107777 A ,B | 01-06-1988 |
| | | DE 3752167 D1 | 12-03-1998 |
| | | DE 3752167 T2 | 25-06-1998 |
| | | DE 3752201 D1 | 13-08-1998 |
| | | DE 3752201 T2 | 17-12-1998 |
| | | DE 3789536 D1 | 11-05-1994 |
| | | DE 3789536 T2 | 18-08-1994 |
| | | DK 131893 A | 24-11-1993 |
| | | DK 144295 A | 19-12-1995 |
| | | DK 575887 A | 14-05-1988 |
| | | EP 0268956 A2 | 01-06-1988 |
| | | EP 0475456 A1 | 18-03-1992 |
| | | EP 0654471 A1 | 24-05-1995 |
| | | EP 0786461 A1 | 30-07-1997 |
| | | ES 2061471 T3 | 16-12-1994 |
| | | ES 2112260 T3 | 01-04-1998 |
| | | ES 2118457 T3 | 16-09-1998 |
| | | FI 874709 A ,B, | 14-05-1988 |
| | | GR 3026762 T3 | 31-07-1998 |
| | | HK 194296 A | 01-11-1996 |
| | | HU 45524 A2 | 28-07-1988 |
| | | JP 2544567 B2 | 16-10-1996 |
| | | JP 5247035 A | 24-09-1993 |
| | | JP 2576843 B2 | 29-01-1997 |
| | | JP 7291967 A | 07-11-1995 |
| | | JP 1006270 A | 10-01-1989 |
| | | JP 1953321 C | 28-07-1995 |
| | | JP 6074272 B | 21-09-1994 |
| | | KR 9103330 B1 | 27-05-1991 |
| | | LU 90396 A9 | 07-07-1999 |
| | | NO 874477 A ,B, | 16-05-1988 |
| | | NZ 222488 A | 26-05-1993 |
| | | US 5840910 A | 24-11-1998 |
| | | US 5998445 A | 07-12-1999 |
| | | DD 290192 A5 | 23-05-1991 |
| | | SU 1780535 A3 | 07-12-1992 |
| | | RU 2035461 C1 | 20-05-1995 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 00 4420

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2004

| Patent document cited in search report | | Publication date | Patent family member(s) | | | Publication date |
|---|---|---|---|---|---|---|
| WO 0214286 | A | 21-02-2002 | AU | 8476301 | A | 18-02-2002 |
| | | | AU | 8488001 | A | 25-02-2002 |
| | | | CA | 2419314 | A1 | 21-02-2002 |
| | | | CZ | 20030668 | A3 | 14-04-2004 |
| | | | EP | 1317434 | A1 | 11-06-2003 |
| | | | HU | 0302874 | A2 | 29-12-2003 |
| | | | JP | 2004506622 | T | 04-03-2004 |
| | | | WO | 0212200 | A1 | 14-02-2002 |
| | | | WO | 0214286 | A1 | 21-02-2002 |
| | | | US | 2002115672 | A1 | 22-08-2002 |
| | | | US | 2002115673 | A1 | 22-08-2002 |
| WO 03082858 | A | 09-10-2003 | WO | 03082858 | A1 | 09-10-2003 |

EPO FORM P0459

For more details about this annex : see Official Journal of the European Patent Office, No. 12/82